# EUROPEAN PATENT SPECIFICATION

(11) **EP 2 512 679 B1**
(45) Date of publication and mention of the grant of the patent: **10.06.2015**
(21) Application number: 10812888.5
(22) Date of filing: 15.12.2010
(51) Int. Cl.: B01L 3/14, A61B 10/00

(54) **TEST TUBE FOR DIAGNOSTIC ANALYSES**
REAGENZGLAS FÜR DIAGNOSTISCHE ANALYSEN
TUBE À ESSAI POUR ANALYSES DE DIAGNOSTIC

(30) Priority: 15.12.2009 IT UD20090228
(43) Date of publication of application: 24.10.2012
(73) Proprietor: Alifax Holding S.p.A., 35020 Polverara (PD) (IT)
(72) Inventor: GALIANO, Paolo, I-35100 Padova (IT)
(74) Representative: Petraz, Davide Luigi
(86) International application number: PCT/IB2010/003243
(87) International publication number: WO 2011/073768

(56) References cited:
- EP-A1- 0 298 513
- DE-A1- 19 718 081
- DE-U1- 9 418 060
- FR-A3- 2 575 652
- US-A- 2 722 257
- US-A- 4 215 700
- US-A- 4 250 893
- US-A- 5 261 881

## Description

### FIELD OF THE INVENTION

The present invention concerns a test tube usable in laboratory diagnostics for collecting urine samples or biological expectorates, synovial, pleural, cerebrospinal, ascitic, peritoneal, tracheal or bronco-alveolar fluids, saliva or diluted feces, in order to carry out bacteriological, microbiological and chemical examinations.

### BACKGROUND OF THE INVENTION

It is known that laboratory diagnostics uses traditional containers, like vases, suitable for the collection of the primary sample, which is then transferred into plastic or glass test tubes, to collect biological samples of various type and possible containing anti-coagulants or stabilizers.

Vacuum test tubes are also known, in which, thanks to the internal depression, a predetermined quantity of the desired sample is taken in, to prevent contact with air and hence to guarantee the sterility of the sample taken.

The transfer of the sample taken by the specialized personnel into the test tubes is normally carried out manually, by means of pipettes, among which the "Pasteur" pipettes are known, or by means of automatic sampling and dispensing systems of the native sample, which transfer everything to an analyzing machine. The Pasteur pipette is formed by a little tube of thin plastic, with an aperture in the lower part and on the top of which, wider than the lower part, there is a teat made of the same material as the test tube body. Pressing the teat causes the air contained in the tube to come out. Keeping the teat pressed, the tube is immersed into the liquid and, releasing the pressure, a pressure imbalance is caused by means of which the liquid rises along the tube and is not able to exit until the teat is pressed again. However, the Pasteur pipette cannot be used as a precision instrument because it has no graduated scale, and also it cannot be used directly inside the diagnostic analysis instrument.

In this context, due to the ever-growing need to perform a high number of analyses in a short period of time and with great reliability, in the field of diagnostic analysis there is a continuous search for simplification in the activities performed, and a simultaneous increase in productivity, in order to speed up operations but also to reduce repetitions and errors.

Consequently, it is required for example that actions that are repeated in a large number, such as the taking of samples and the sealing of the test tube, must be simplified to the utmost while guaranteeing absolute reliability.

From the documents US-A-2,722,257, US-A-4,250,893, US-A-5,261,881, US-A-4,215,700 and DE-U-9418060 test tubes are known to collect and contain a sample for the purposes of diagnostic analysis, which comprise a containing body, a sampling member, suction means and a closing element.

Purpose of the present invention is to achieve a test tube that can be used easily to collect a sample made directly by the patient, such as urine or saliva samples, or by the doctor responsible for sampling, in order to carry out microbiological or bacteriological analyses, without making any other transfers into subsequent and dedicated test tubes that then have to be housed in the specific instrumentation in order to make the diagnostic analyses directly, thus allowing to simplify and speed up the sampling operations, the operations of closing the test tubes and possibly attaching them to the analysis machine, and also of re-opening them in order to carry out the sampling required in the same test tube where the initial sample was collected, so as to be able to start the step of dispensing the relative content into automatic instruments or supports suitable for the examination required.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purpose, a test tube for taking and containing a biological sample of various types, or other liquid or similar substance, for diagnostic analyses is provided according to claim 1.

According to the present invention, the tubular member is conformed to be selectively removable so as to allow the closing element to pass into the superimposed position, to close the aperture of the test tube.

In particular, the support element is connected to the closing element by means of a flexible connection element configured to deform when the closing element is taken to the position where it closes the aperture, and the tubular member is configured to detach from the support element when the closing element is taken from a position outside the bulk of the containing body in the closed position.

In the invention, the closing element is configured to interfere, in its passage to said superimposed position, with the tubular member, determining the detachment thereof from the test tube, in some forms of embodiment by means of a predefined breakage and in other forms of embodiment by means of uncoupling.

In some forms of embodiment, the tubular member has at least a line of weakening in correspondence with the region where it connects to the closing device, which line defines a condition of preferential breakage for the selective detachment of the tubular member from the closing device.

According to a variant, the line of weakening develops along a closed path so as to define, advantageously, a complete detachment of the tubular member from the closing device.

Some forms of embodiment of the present invention provide that the sampling tubular member is coupled with the support element of the closing device by means of a coupling portion that has the line of weakening.

In this way, after facilitating the operation of filling the test tube, the sampling tubular member can easily be removed by means of the same operation to close the test tube.

In the invention, the support element is coupled or constrained to the end edge and connected to the closing element. The tubular member is made or disposed on the support element of the closing device. According to a variant, the tubular member is made in a single body, or is coupled with the support element, from which it protrudes toward the outside, so as to be interposed between the support element and the closing element, in its passage to said superimposed position.

In some forms of embodiment, the closing element is also movable to a position outside the bulk of the containing body of the test tube, in particular of the support element. In some forms of embodiment of the invention, the closing element can be positioned, outside the bulk of the support element, on a theoretical lying plane common to the support element.

According to the present invention, the flexible connection element is selectively deformable, or bendable, to allow the closing element to move from a position outside the bulk of the support element to a superimposed position above the bulk of the support element so as to close the aperture of the test tube, and vice versa.

In some forms of embodiment, the closing element is conformed so as to be able to be coupled, in the superimposed position, with the support element so as to close completely or seal the containing body, advantageously under pressure.

Advantageously, therefore, the closing element of the closing device can have a double function, that of breaking the sampling tubular member and of closing the containing body hermetically. With the closing device according to the invention, the closing operation is very simple and quick, guaranteeing the necessary hermetic seal after sampling, thanks to the closing element.

In one form of embodiment of the present invention, the suction means are formed by at least an elastically deformable part of the containing body, which is conformed to be selectively compressed from a first inactive position to a second deformed position, so as to expel a determinate volume of air from the containing body and expand again, once the compression is no longer applied, returning to the first inactive position, so as to pick up, by means of a suction effect, a corresponding volume of sample taken in through the tubular member.

The present invention therefore allows to achieve a test tube for liquid or diluted biological samples, which is easily used by the patient himself or by a technical operator, both at the moment of primary sampling, and in sealing, and also in the course of all the subsequent analysis operations.

For chemical analyses it is normal procedure to insert reactive strips in the urine sample. The strips contain a desired number of chemical pads, on average eleven, which detect the required substances. The operating steps that comprise the opening of the cap, the insertion of the strip and the closing of the cap according to the present invention allow to have the reactive part of the strip in contact with the urine in the test tube while the end of the inert strip protrudes from the closed cap. By mixing or stirring the test tube, it is possible to contact the urine sample with all the pads deposited on the nitrocellulose strip. Reopening the cap allows the strip to be removed manually and then read in the appropriate instruments. The test tube according to the present invention can therefore be conveniently inserted in automatic instruments for the strip reading analysis.

With the test tube according to the invention, the samples can be taken and sealed sequentially and simply, for the purposes of the subsequent analysis or preservation. The sampling test tube itself can therefore become the test tube used for the examination, by inserting it directly into an analysis instrument.

In fact, with the invention it is possible to obtain a sealed test tube right from the moment the sample to be analyzed is taken, by means of an easy operation of sealing and closing made by the patient himself or the responsible operator.

The sealed test tube is therefore ready for use in analysis using automatic instruments or manual methods without needing other transfers of the initial sample taken.

This also has the advantage that no bad or unpleasant odors are released into the environment, since the test tube is sealed immediately.

According to another form of embodiment of the invention, the elastically deformable part is formed by at least a deformable portion of at least a lateral and/or bottom wall of the containing body. This solution has the advantage that it is sufficient to make a simple and easy action of lateral or lower pressure, also using the finger of one hand, by the operator or patient, in order to take the sample.

According to another form of embodiment, the elastically deformable part is formed by a concertina-type pumping member which forms at least part of the bottom of the containing body. This embodiment has the advantage, apart from its intuitive and easy drive, that it gives a precise dosing of the sample taken.

In some forms of embodiment of the invention, the tubular member is made as a single piece with the closing device and can be detached by means of a predefined breakage by acting on the closing element, pressure with the fingers or other.

Alternative forms of embodiment provide, instead, that the tubular member is made as a separate body from the closing device or the containing body of the test tube, and that it is coupled, in a selectively removable manner, by means of jointing, anchorage or other clamping, with a relative portion of the closing device or the containing body.

It is clear that, in some uses of the present invention, the tubular member can also be removed by means of an action of lateral pressure with the fingers, or using a breaking edge, with a knurled or other shape, of a possible container for collecting medical waste.

Some forms of embodiment of the invention provide that the closing device is made in a single piece, since the sampling tubular member has a structural and material continuity at least with the closing element.

In a variant embodiment the closing device is a single body made by means of a single extrusion and molding operation.

In some variant embodiments, on the contrary, the closing device is formed by two separate parts, of which the support element is coupled to the end edge and the closing element is separate and can be made to close directly by the user.

In some forms of embodiment, the sampling tubular member is a separate component from the closing device. In this case, the tubular member can be coupled, anchored or jointed with the support element of the closing device or other portion of the containing body, for example it can be jointed inside the containing body of the test tube, during the assembly step, by means of a double anchoring ring inside the test tube which defines an annular coupling seating for a base portion of the tubular member.

In such forms of embodiment, the tubular member is easily removable from its anchored position in the test tube, for example by making a simple lateral pressure with the fingers. The detachment of the tubular member from the body of the test tube or from the closing device is thus made simple and intuitive and allows a subsequent closure with the closing element.

According to one form of embodiment, the containing body has centering and clamping elements made in a single piece with the containing body. These elements allow the test tube to be inserted easily and securely in suitable housings of a rack, loaders, trays or other transport members for the test tubes of an analysis machine, where it is easily clamped. Furthermore, these elements function as a precise obligatory application zone for labels, bar codes or other identifications. The reliable clamping of the test tube in the analysis machine is also advantageous for the correct reading of the bar codes, identification labels or RFID tags applicable on the test tubes.

In an advantageous variant solution the containing body is a single body made by means of a single extrusion and molding operation of plastic polymer material, for example polyethylene.

In other forms of embodiment, as mentioned above, the containing body can be formed by two pieces suitably jointed together, where a first has a containing function and the second functions as a sampling tubular member. In these embodiments, the tubular member can be jointed to the containing body by means of an internal cantilever consisting of two rings in relief, such as to accommodate a base portion of the tubular member. This solution allows the tubular member to be easily removed by means of a simple pressure with the fingers with respect to the vertical position of the tubular member, also made directly by the patient. In this way, the test tube can be closed and delivered directly sealed to the health workers. The invention thus obtains rapidity and safety in the sampling of urine or other types of sample, preventing the annoying re-openings of known containers which would normally release into the air bad smells of organic components. The test tube according to the invention can in fact be housed directly in the instruments used for the examinations required, which are provided with a perforating needle suitable to pick up the quantity of sample to be analyzed without having to be re-opened.

In some forms of embodiment, along the containing body the test tube has a space, surface or housing to position a suitable label with bar code, or RFID tag, identifying the patient, or can be provided, from the moment it is made, with bar code labels with non-repeatable numbers or RFID tags.

This function allows a unique coupling of the bar code, or RFID tag, and the patient, from the moment it is delivered to the patient by the health worker.

The test tube according to the present invention is able to contain quantities of sample varying for example from 1 ml to 15 ml and allows to load desired fractions, for example 3 ml, 6 ml, etc., of the same sample, by means of a simple pressure on the compressible part of the test tube, taking care to expel the air with the tubular member in a vertical position, before every fraction of sample.

The test tube is suitable to take and contain biological samples such as urine, bronco-alveolar lavage, tracheal aspirate, liquid expectorate, synovial, pleural, cerebrospinal, ascitic and peritoneal fluid, diluted feces, saliva or other liquid or similar substances, by means of a simple and rapid pressure operation on the elastically deformable and compressible part by using one or two fingers of the operator or even the patient himself.

The test tube according to the present invention, apart from the sample, can also contain liquid substances or powders, such as for example bacterial stabilizers, such as boric acid, for the urine. For example, with the present invention the boric acid can be measured in the exact volumetry of the desired ratio of boric acid/urine.

At the moment when pressure is exerted on the elastically deformable and compressible part, the concertina pump on the bottom of the test tube or on the lateral wall, there will be no leakage of liquid or powder possibly present as anticoagulant or chemical additives possibly inserted, provided that it is kept in a vertical position by the patient or the operator.

The present invention allows to perform the sampling operation by the patient himself, for example in the case of a urine sample, and to deliver to the laboratory personnel a test tube filled with the necessary quantity, perfectly sealed and advantageously labeled with a bar code, without any intervention of the staff.

The invention gives a considerable saving in time for the health workers and can prevent errors in identifying the bar code or other label, since it can be applied to the test tube by the patient himself.

The sampling test tube thus sealed becomes the work test tube and is inserted into the normal sample racks for automatic instruments. Once inserted in the rack, the closing element of the test tube can be easily opened and closed with traditional automatic or manual "de-capping" devices.

In some forms of embodiment, the openable and closable closing element allows to speed up the operation of sucking up the sample by the sampling needle of the analysis machine, since it might not need any perforation of the rubber cap commonly used in traditional test tubes and, especially if compared with vacuum test tubes, it does not require a perforating force of many kilograms to be exerted.

If the sample for examination is taken by a perforating needle, apart from the ease of sampling, there is also the advantage that no volatile substances are expelled, such as bad or unpleasant odors of the biological material processed.

Another advantage of the invention is the overall cost of the test tube according to the invention, which is much less than known test tubes on the market.

Another feature of the present invention concerns a method for analyzing liquid or diluted biological samples which provides, in a sequential manner, to introduce the sample taken from a patient into a test tube using an introduction portion of the test tube itself and to seal the test tube by means of a closing device of the test tube for subsequent analysis or preservation, in which the sealed test tube is used directly also as a container in the analysis by means of automatic instruments or using manual methods, without needing other transfers of the initial sample taken.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a three-dimensional view of a test tube according to the present invention;
- fig. 2 is a lateral view of the test tube in fig. 1;
- fig. 3 is a three-dimensional view of a variant test tube according to the present invention;
- fig. 4 is a lateral view of the test tube in fig. 3;
- fig. 5 is a plane view from above of a part of the test tube according to the present invention;
- fig. 6 is a section from VI to VI of fig. 5;
- fig. 7 is a representation of a variant of the test tube according to the present invention;
- fig. 8 is a schematic representation of a sampling operation using the test tube according to the present invention;
- fig. 9a is a schematic representation of a closing step of the test tube according to the present invention;
- fig. 9b is a representation in a closed condition of the test tube according to the present invention;
- fig. 10a is a schematic representation of in a re-opened condition of the test tube according to the present invention;
- fig. 10b is a schematic representation of a sampling operation using a needle by the test tube according to the present invention.

To facilitate comprehension, the same reference numbers have been used, where possible, to identify identical common elements in the drawings. It is understood that elements and characteristics of one form of embodiment can conveniently be incorporated into other forms of embodiment without further clarifications.

### DETAILED DESCRIPTION OF A PREFERENTIAL FORM OF EMBODIMENT

With reference to the attached drawings, a test tube 10, 110 according to the present invention is usable in laboratory diagnostics to take desired quantities of a sample of a liquid type, biological or similar, such as for example urine or other, for the purposes of a specific analysis, or other liquid substance, reagent or other, used in the analyses.

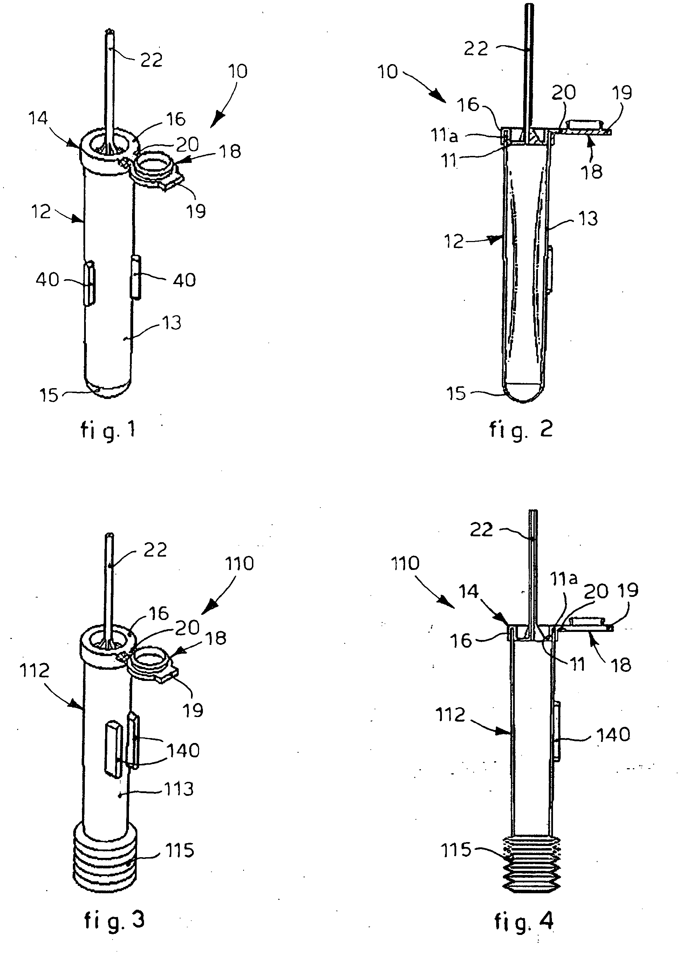

According to a first form of embodiment of the present invention, shown in figs. 1 and 2, the test tube 10 comprises a containing body 12 hollow inside, made of moldable plastic material, for example polyethylene.

The containing body 12 has a traditional shape, elongated or oblong, typical of laboratory test tubes and provides an upper end edge 11 a, typically circular, which delimits an upper aperture 11 to introduce the sample inside.

The containing body 12 also provides a central part defined by a cylindrical wall 13 and a lower closing bottom 15 (figs. 1 and 2), usually rounded, beveled or curved.

The test tube 10 comprises a closing device 14 that is constrained to the end edge 11a of the aperture 11 of the containing body 12. The coupling is advantageously effected by means of jointing during the production of the test tube 10.

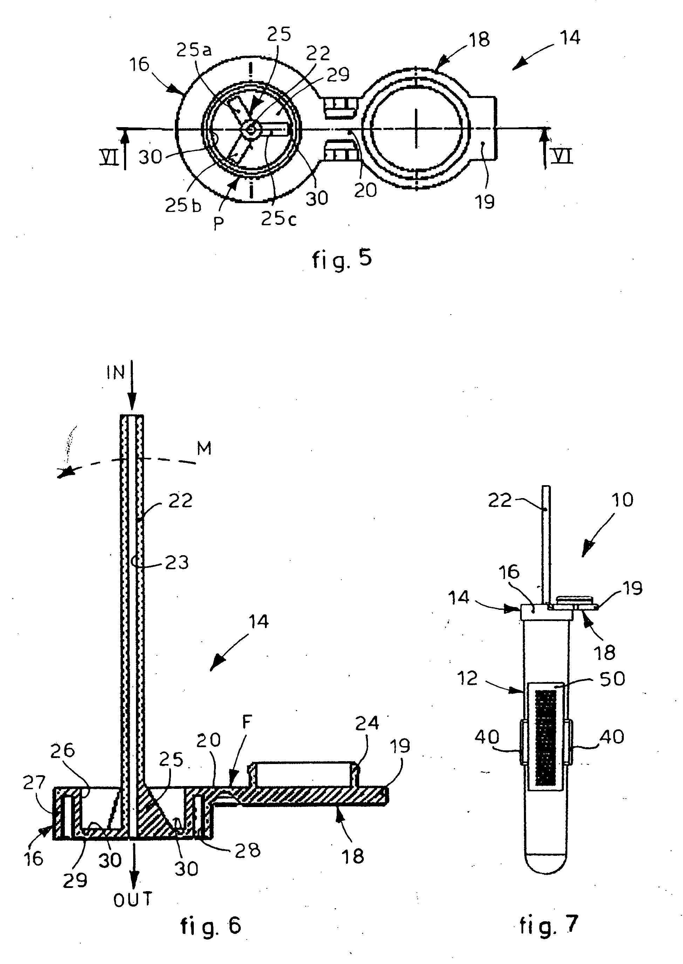

The closing device 14 comprises, in particular, a support element 16 that supports a sampling rod or spout 22, integrated therewith, a closing element 18, for example a so-called flip cap, normally in a position outside the bulk of the support element 16, and a flexible connection element 20, deformable or bendable, which functions as a connection between the support element 16 and the closing element 18 (figs. 5 and 6).

The flexible connection element 20 can be bent to allow the passage of the closing element 18, which is rotated by 180°, from its external position, in fig. 6 shown for example horizontal, to a closed position superimposed above the support element 16, to be coupled with it under pressure and seal the containing body 12.

The cylindrical wall 13 of the test tube 10 is made with a desired reduced thickness of a substantially central portion thereof, such as to allow a certain elastic deformation and compressibility.

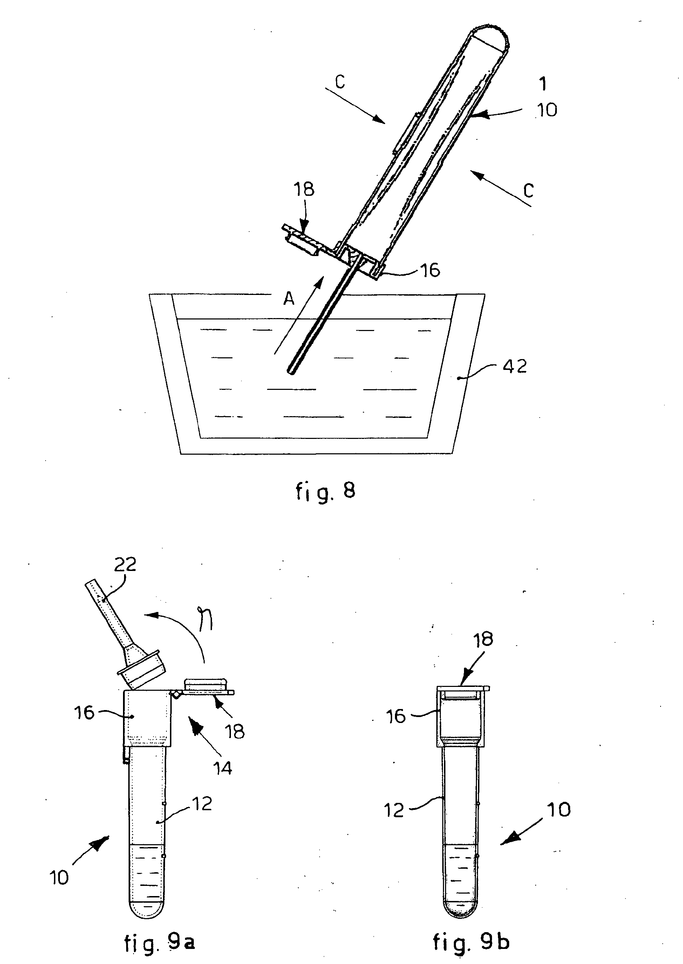

Consequently, the cylindrical part 13 can be used as a pumping and suction member, using manual pressure, in this case with a force substantially radial to the test tube (fig. 8), by an operator or the patient, which expels the air, or part of the air, contained inside it, to obtain a vacuum suction of a portion of the sample equivalent to the volume of air expelled during the compression by pressure with the fingers.

The operator or the patient, in particular, presses the cylindrical wall 13 to compress it from a first normal inactive position to a second, compressed position and to create a suction effect, correlated to the precise portion of sample to be taken in, for example 3 ml or 6 ml. The rod 22 is immersed in a container with the sample to be taken and the pressure exerted is released, to take in a desired portion from the container, as can also be seen in figs. 8, 9a and 9b.

Subsequently, if a second portion of sample is to be taken, if the first volume was not sufficient, the test tube 10 is rotated on itself by 180° vertically, that is, with the rod 22 facing upward to prevent leakages, and then, again exerting a pressure with the fingers on the cylindrical wall 13, the internal air is expelled, the rod 22 is again immersed in a container, the same or a different one according to requirements, which contains a certain quantity of sample to be taken or a reagent, a stabilizer or other.

At this point, the pressure on the cylindrical wall 13 is again released, and this passes again to the first inactive position. Due to the effect of the vacuum suction, the determinate portion of sample is taken in through the rod 22, inside the containing body 12. The test tube 10 is then rotated again vertically by 180° with the rod 22 facing upward, the rod 22 is removed from the support element 16 and the test tube 10 is then re-closed with the closing element 18.

With reference again to the closing device 14, the support element 16 comprises a lateral wall 27 with a toroidal development, with a shape mating with that of the aperture 11 and which delimits a central cylindrical seating 26, and a bottom 29, made in structural and material continuity with the lateral wall 27.

The support element 16 is constrained to the upper portion, that is, the end edge 11 a of the cylindrical wall 13 that delimits the aperture 11 of the test tube 10 and, for this purpose, on the side normally facing the inside of the containing body 12, has an annular insertion seating 28 made axially along the lateral wall 27 and mating with the shape of the cylindrical wall 13 (fig. 6).

The rod 22 of the support element 16 has a substantially linear oblong development, like a pipette or perfusion tube. In some forms of embodiment, it can have a length varying from a few centimeters to 12 cm, although these measurements must not be understood as restrictive of the field of protection of the present invention. The rod 22 is hollow inside, to define a through sampling tube 23 with a mainly longitudinal development, which determines a sampling path for the sample from an inlet, indicated by IN in fig. 6, to an outlet, indicated by OUT in fig. 6. The OUT outlet normally faces toward the inside of the containing body 12 when the support element 16 is coupled with the aperture 11.

The rod 22 couples, with a coupling portion 25, on the bottom 29 of the support element 16 and is also made with a structural and material continuity with the lateral wall 27 and the bottom 29. The structural and material continuity is advantageously obtainable by means of a single extrusion and molding operation of a plastic material, for example polyethylene, or in any case the same material that makes up the containing body 12, by means of which the whole support element 16 is made, including the rod 22, the lateral wall 27 that delimits the cylindrical seating 26, and the bottom 29, and the closing element 18.

The coupling portion 25 of rod 22 comprises three arms 25a, 25b, 25c, in this case disposed at 120° with respect to each other, which diverge toward the bottom 29 where one or two lines of weakening 30 (fig. 5) are provided, made on the bottom 29 to determine a predefined breakage. The lines of weakening 30 are also advantageously made in the course of the extrusion and molding operation.

The lines of weakening 30 are shaped and sized during the design stage so as to break, if subjected to the appropriate stress, and to cause the rod 22 to detach from the bottom 29 of the support element 16. If the line of weakening 30 has a closed profile P (fig. 5), the rod 22 is completely detached. According to another variant, an open profile of the line of weakening 30 allows a partial detachment of the rod 22.

In particular, detachment is obtained when the rod 22 is subjected to a force of an appropriate value, direction and sense, for example a lateral pressure or a flexion moment applied by the operator or patient on the central or upper portion of the rod 22, for example in an anti-clockwise direction with reference to the arrow F in fig. 6.

The configuration at 120° of the arms 25a, 25b, 25c allows an optimum distribution of the stress applied to the rod 22, so as to define a precise and reliable detachment.

Once the containing body 12 has been filed with the desired quantity of sample, for example 3 ml or 6 ml, as explained above, the test tube 10 is still open, by means of the path along the sampling pipe 23 which connects the inside with the outside, and has to be closed so as to prevent the leakage of the sample, volatile substances or odors connected thereto.

The test tube 10 is conveniently closed by means of the closing element 18.

The closing element 18 is conformed for coupling by pressure with the support element 16 and has an external surface, normally facing upward in its condition outside the bulk of the support element 16, with reference to fig. 6, from which a collar element 24 extends.

The collar element 24 has sizes mating with those of the cylindrical seating 26 of the support element 16, to achieve therewith a same-shape coupling.

The rotation by 180° of the closing element 18 around a fulcrum, indicated by F in fig. 6, and defined by the substantially central part of the flexible connection element 20, causes the collar element 24 to find itself facing downward and the closing element 18 to overlap with the support element 16. This allows to insert the collar element 24 in the cylindrical seating 26 so as to close the test tube 10 hermetically.

At the same time, the sampling rod 22 is removed from the support element 16, so as not to be an obstacle to the overlapping of the closing element 18 and the closing of the test tube 10. This can advantageously happen thanks to the same rotation movement of the closing element 18 which determines the interference of the closing element 18 with the rod 22 which, applying the necessary lateral pressure, breaks along the lines of weakening 30 and can be removed.

Consequently, the closing element 18 can have the double function of sealing the test tube 10, after sampling, and breaking the rod 22 so it can be removed.

The closing element 18 also has, on its periphery, in this case in a position diametrically opposite the flexible connection element 20, a tooth or other protrusion 19, able to be driven laterally with a cantilevered movement by a de-capping member, automatic or manual, to remove the closing element 18 and effect the desired analysis in the diagnostic analysis machine. The closing element 18 is removed from the support element 16, but it remains always connected to the test tube 10 thanks to the flexible connection element 20.

Again with reference to the containing body 12, the cylindrical wall 13 has attachment and centering pins 40 in an asymmetrical position, suitable for vertical insertion and/or bayonet-type attachment in a rack of the analysis machine, as described for example in the Italian patent application UD2008A000128, in the name of the present Applicant.

Figs. 3 and 4 show a second form of embodiment, indicated by the reference number 110, of the test tube according to the present invention, which proposes an alternative to determine the vacuum suction effect of the sample.

In the test tube 110, the containing body, indicated by the reference number 112, has a central cylindrical wall 113, which unlike the cylindrical wall 13 of the test tube 10, is of a substantially rigid type or in any case which is not compressible for suction purposes. The bottom of the containing body 112 consists instead of a concertina-type pumping member 115, or a hollow member shaped with contiguous pleated surfaces, made of flexible and elastically deformable material along a desired deformation travel associated with a determinate volume of air, between an inactive position and a compressed or flattened position. The concertina-type pumping member 115 may be pressed, in this case by applying a force in a direction axial with respect to the test tube 110, to expel the volume of air normally contained inside it, and to obtain a vacuum suction effect with a function similar to that of the test tube 10. As already described above, the test tube 110 is also rotated vertical by 180° and the rod 22 is immersed in the sample to be taken. Once the pressure on the concertina-type pumping member 115 is released, a quantity of sample equal to the volume of the concertina-type pumping member 115 will be taken in through the rod 22. Again, the test tube 110 is rotated vertical by 180° and closed by the closing element 18 which breaks the rod 22. This second form of embodiment of test tube 110 has the advantage of an accurate calibration of the quantity of sample taken, since the quantity is directly correlated to the compression travel of the concertina-type pumping member 115 and therefore can easily be obtained, in practice, by an operator or the patient himself.

To facilitate sampling accuracy, the test tube 10, 110 may provide notched level indicators or other demarcation lines along the containing body 12, 112, to verify the correct volumetric filling. The test tube 110 is shown in fig. 3 with centering pins 140 disposed symmetrical and suitable to be inserted frontally into the rack of the analysis machine.

In some forms of embodiment the test tube 110 could be provided with asymmetrical centering pins 40 instead and also, in other forms of embodiment, the test tube 10 could be provided with symmetrical centering pins 40.

Both the test tube 10 and the test tube 110 allow to apply, in the space comprised between the centering pins 40, 140, a bar code or an RFID tag, used to recognize, trace and localize the test tube, the sample contained and the analysis to be carried out. The solution with centering pins 40, 140 is advantageous to this end, because they are disposed at a distance, one from the other, compatible with the sizes of the bar code or RFID tag, defining a relative obligatory application position and hence repeatable without errors, as can be seen for example in fig. 7.

In some forms of embodiment of the sampling and diagnostic analysis, the test tube is used in accordance with the following steps (figs. 8, 9a, 9b, 10a and 10b), for example for urine samples. The patient collects his urine in a plastic glass 42 and normally the urine content would be transferred by the technical operators into containers or test tubes suitable to be accommodated in the specific instruments and, in the case of normal test tubes, that is, not vacuum, left open. With the present invention, on the other hand, the patient or the health worker takes the urine sample, or other biological sample, directly from the glass 42 by means of the test tube 10 which is then sealed and used, without any other transfers, in the subsequent analysis steps as analysis container. In this way, the propagation of bad or unpleasant odors from the sample in the test tube is also prevented.

The initial urine sample, collected in the glass 42 by the patient, is taken in using the test tube 10 according to the present invention through the sampling rod 22, which will not contaminate the sample taken. In particular, the patient presses, in this case radially as indicated by the arrow C or axially in the solution with the concertina portion 115, on the containing body 12 so as to obtain the suction effect upward as indicated by arrow A (fig. 8).

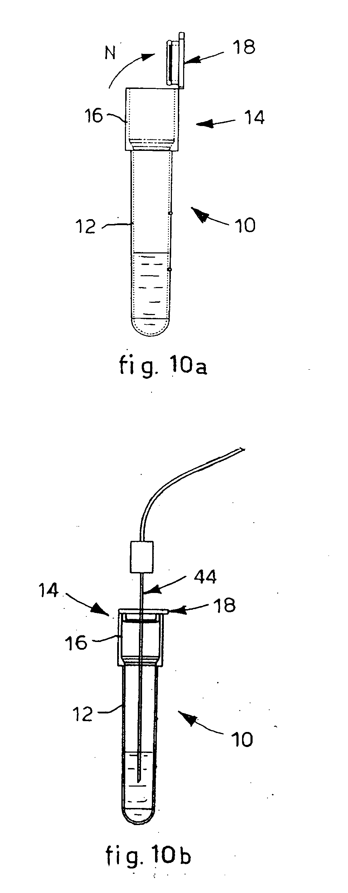

The test tube is then closed by the patient who, with a simple rotation as indicated by arrow M in fig. 9a and pressure of the closing element 18 on the intake rod 22, determines the breakage thereof and hence the closing and sealing of the test tube (fig. 9b). The same test tube 10 is therefore delivered closed to the reception operator, who will be able to attach the bar code or patient identification label or write the data of the examination required. The test tube 10 can be inserted in trays or racks or loaders of an automated analysis instrument. As can be seen in fig. 10a, the test tube 10 can be opened easily, thanks to a rotation of the closing element 18 as indicated by the arrow N, so as to dispense manually a desired portion of the sample to be analyzed. As can be seen in fig. 10b, the portion to be analyzed can be easily taken automatically with a controlled sampling needle 44 of an automated analysis instrument, according to some forms of embodiment, by perforating the closing element 18 with the needle 44 or, according to other forms of embodiment, by opening the closing element 18 as in fig. 10a and inserting the needle 44 through the aperture 11.

Advantageously, therefore, the step of transferring urine into subsequent test tubes is eliminated, inasmuch as the test tube 10 according to the invention becomes the test tube which will be loaded into the appropriate instrument or used for manual microbiological doses. In the case of repeated analyses or further controls, the same test tube 10 can be opened again, as in fig. 10a, by the technical operators or by automatic de-capping devices present in the instruments.

It is clear that modifications and/or additions of parts may be made to the test tube as described heretofore, without departing from the field and scope of the present invention.

It is also clear that, although the present invention has been described with reference to some examples, a person of skill in the art shall be able to achieve equivalent forms of test tube, having the characteristics as set forth in the claims and hence all coming within the field of protection defined thereby.

## Claims

1. Test tube for taking and containing a sample for diagnostic analyses, comprising:
- a containing body (12) with an end edge (11a) that delimits an aperture (11);
- a tubular member (22) protruding toward the outside with respect to said end edge (11a) and connected with the inside of said containing body (12) through said aperture (11) for the passage of the sample;
- suction means (13, 115) made in a single piece with said containing body (12) and selectively drivable to take in a desired volume of sample through said tubular member (22);
- a closing device (14) constrained to said end edge (11a) and comprising a closing element (18) selectively movable toward a superimposed closing position located above said end edge (11a) in order to close said aperture (11) and a support element (16) for said tubular member (22), coupled to said end edge (11a) and connected to the closing element(18), wherein in said superimposed closing position, said closing element (18) is conformed so as to be coupled with said support element (16) so as to close said containing body (12) completely, **characterized in that** said support element (16) is connected to said closing element (18) by means of a flexible connection element (20), configured to deform when the closing element (18) is taken to said closing position to close the aperture (11), said tubular member (22) being interposed between said support element (16) and said closing element (18), said tubular member (22) being configured to detach from said support element (16) when the closing element (18) is moved from a position outside the bulk of the containing body (12) to said closing position.

2. Test tube as in claim 1, **characterized in that** the detachment of the tubular member (22) is provided by uncoupling.

3. Test tube as in claim 1, **characterized in that** said tubular member (22) has at least a line of weakening (30) in correspondence with the region where it connects to the closing device (14), which defines a preferential breaking condition for the selective detachment of said tubular member (22) from said closing device (14).

4. Test tube as in claim 3, **characterized in that** said line of weakening (30) develops along a closed path, so as to define a complete detachment of the tubular member (22) from said closing device (14).

5. Test tube as in claim 3 or 4, **characterized in that** the tubular member (22) couples on the support element (16) of said closing device (14) by means of a coupling portion (25) that has said at least one line of weakening (30) of preferential breakage.

6. Test tube as in any claim hereinbefore, **characterized in that** said suction means are formed by at least an elastically deformable part (13, 115), which is conformed to be selectively compressed from a first inactive position to a second deformed position, so as to expel a determinate volume of air from the containing body (12) and to expand again, returning to said first inactive position, so as to pick up the desired volume of sample by means of the suction effect.

7. Test tube as in claim 6, **characterized in that** said elastically deformable part is formed by at least a deformable portion of at least a lateral (13) or bottom wall of said containing body (12).

8. Test tube as in claim 6, **characterized in that** said elastically deformable part is formed by a concertina-type pumping member (115) which forms at least part of the bottom of said containing body (12).

9. Test tube as in any claim hereinbefore, **characterized in that** the tubular member (22) is made in a single piece with the closing device (14).

10. Test tube as in any claim hereinbefore, **characterized in that** the tubular member (22) is made as a separate piece with respect to the closing device (14) or the containing body (12) and is coupled in a selectively removable manner, by means of jointing, anchoring or other clamping, with a relative portion of the closing device (14) or containing body (12).

11. Test tube as in any claim hereinbefore, **characterized in that** said tubular member is conformed as a sampling spout or rod (22).

12. Test tube as in any claim hereinbefore, **characterized in that** said closing element (18) has a peripheral tooth (19) able to be driven with a cantilevered movement for opening/closing.

13. Test tube as in any claim hereinbefore, **characterized in that** said closing device (14) is made in a single piece, said tubular member (22) being made as a structural and material continuation at least of said closing element (18).

14. Test tube as in claim 13, **characterized in that** said closing device (14) is a single piece made by means of a single extrusion and molding operation.

15. Test tube as in any claim hereinbefore, **characterized in that** said containing body (12) includes centering and clamping elements (40, 140) made in a single piece with said containing body (12).

16. Test tube as in any claim hereinbefore, **characterized in that** said containing body (12) is a single piece made by means of a single operation of extruding and molding plastic polymer material, also including the making of said suction means (13, 115).

17. Method for analyzing liquid or diluted biological samples which provides, sequentially, to introduce the sample taken from a patient into a test tube (10) as in any claim hereinbefore, using an introduction portion (22) of the test tube (10) and to seal said test tube by means of a closing device (14) of the test tube (10) for subsequent analysis or preservation, wherein said sealed test tube (10) is also used directly as a container in the analysis by means of automatic instrumentation or using manual methods, without needing any further transfers of the initial sample.

## Patentansprüche

1. Reagenzglas zum Aufnehmen und Enthalten einer Probe für diagnostische Analysen, umfassend:
- einen Behälterkörper (12) mit einem stirnseitigen Rand (11a), der eine Öffnung (11) eingrenzt;
- ein rohrförmiges Element (22), das mit Bezug auf den stirnseitigen Rand (11a) nach außen vorsteht und mit dem Inneren des Behälterkörpers (12) durch die Öffnung (11) hindurch zum Hindurchgelangen der Probe verbunden ist;
- Saugmittel (13, 115), die mit dem Behälterkörper (12) einstückig ausgebildet sind und selektiv betreibbar sind, um ein gewünschtes Probenvolumen durch das rohrförmige Element (22) aufzunehmen;
- eine Verschlussvorrichtung (14), die mit dem stirnseitigen Rand (11a) verspannt ist und ein Verschlusselement (18) umfasst, das selektiv zu einer überlagernden Verschlussposition hin beweglich ist, die über dem stirnseitigen Rand (11a) angeordnet ist, um die Öffnung (11) zu verschließen, und ein Trägerelement (16) für das rohrförmige Element (22), das mit dem stirnseitigen Rand (11a) gekoppelt ist und mit dem Verschlusselement (18) verbunden ist, wobei das Verschlusselement (18) in der überlagernden Verschlussposition so angepasst ist, dass es mit dem Trägerelement (16) gekoppelt ist, so dass es den Behälterkörper (12) vollständig verschließt,
**dadurch gekennzeichnet, dass** das Trägerelement (16) mit dem Verschlusselement (18) mittels eines flexiblen Verbindungselements (20) verbunden ist, das dazu ausgebildet ist, sich zu verformen, wenn das Verschlusselement (18) in die Verschlussposition gebracht wird, um die Öffnung (11) zu verschließen, wobei das rohrförmige Element (22) zwischen dem Trägerelement (16) und dem Verschlusselement (18) angeordnet ist, wobei das rohrförmige Element (22) dazu ausgebildet ist, von dem Trägerelement (16) entfernt zu werden, wenn das Verschlusselement (18) von einer Position außerhalb des Hauptvolumens des Behälterelements (12) in die Verschlussposition bewegt wird.

2. Reagenzglas gemäß Anspruch 1, **dadurch gekennzeichnet, dass** das Entfernen des rohrförmigen Elements (22) durch eine Entkopplung vorgesehen wird.

3. Reagenzglas gemäß Anspruch 1, **dadurch gekennzeichnet, dass** das rohrförmige Element (22) mindestens eine Sollbruchlinie (30) in Entsprechung zu dem Bereich aufweist, an dem es mit der Verschlussvorrichtung (14) verbunden ist, die einen bevorzugten Bruchzustand für das selektive Entfernen des rohrförmigen Elements (22) von der Verschlussvorrichtung (14) definiert.

4. Reagenzglas gemäß Anspruch 3, **dadurch gekennzeichnet, dass** die Sollbruchlinie (30) entlang einem geschlossenen Pfad verläuft, um so eine vollständige Entfernung des rohrförmigen Elements (22) von der Verschlussvorrichtung (14) zu definieren.

5. Reagenzglas gemäß Anspruch 3 oder 4, **dadurch gekennzeichnet, dass** das rohrförmige Element (22) mittels eines Kopplungsteils (25), der die mindestens eine Sollbruchlinie (30) eines bevorzugten Bruchs aufweist, mit dem Trägerelement (16) der Verschlussvorrichtung (14) gekoppelt ist.

6. Reagenzglas gemäß einem der vorhergehenden Ansprüche, **dadurch gekennzeichnet, dass** die Saugmittel durch mindestens einen elastisch verformbaren Teil (13, 115) ausgebildet sind, der so angepasst ist, dass er von einer ersten inaktiven Position in eine zweite verformte Position selektiv zusammengedrückt werden kann, um so ein vorbestimmtes Luftvolumen aus dem Behälterkörper (12) herauszudrücken, und sich wieder ausdehnt, wobei er in die erste inaktive Position zurückkehrt, um so mittels des Saugeffekts das gewünschte Probenvolumen aufzunehmen.

7. Reagenzglas gemäß Anspruch 6, **dadurch gekennzeichnet, dass** der elastisch verformbare Teil durch mindestens einen verformbaren Teil mindestens einer Seitenwand (13) oder eines Bodens des Behälterkörpers (12) gebildet wird.

8. Reagenzglas gemäß Anspruch 6, **dadurch gekennzeichnet, dass** der elastisch verformbare Teil von einem faltenbalgartigen Pumpelement (115) gebildet wird, das mindestens einen Teil des Bodens des Behälterkörpers (12) bildet.

9. Reagenzglas gemäß einem der vorhergehenden Ansprüche, **dadurch gekennzeichnet, dass** das rohrförmige Element (22) mit der Verschlussvorrichtung (14) einstückig ausgebildet ist.

10. Reagenzglas gemäß einem der vorhergehenden Ansprüche, **dadurch gekennzeichnet, dass** das rohrförmige Element (22) bezüglich der Verschlussvorrichtung (14) oder dem Behälterkörper (12) als ein eigenes Stück ausgebildet ist und in einer selektiv entfernbaren Weise mittels Fügen, Verankern oder anderer Verspannung mit einem entsprechenden Teil der Verschlussvorrichtung (14) oder des Behälterkörpers (12) gekoppelt ist.

11. Reagenzglas gemäß einem der vorhergehenden Ansprüche, **dadurch gekennzeichnet, dass** das rohrförmige Element als ein Probenentnahme-Stutzen oder -Stab (22) ausgebildet ist.

12. Reagenzglas gemäß einem der vorhergehenden Ansprüche, **dadurch gekennzeichnet, dass** das Verschlusselement (18) einen umlaufenden Zahn (19) hat, der dazu fähig ist, mit einer kragarmartigen Bewegung zum Öffnen/Schließen angetrieben zu werden.

13. Reagenzglas gemäß einem der vorhergehenden Ansprüche, **dadurch gekennzeichnet, dass** die Verschlussvorrichtung (14) einstückig ausgebildet ist, wobei das rohrförmige Element (22) als eine strukturelle und materielle Fortsetzung mindestens des Verschlusselements (18) ausgebildet ist.

14. Reagenzglas gemäß Anspruch 13, **dadurch gekennzeichnet, dass** die Verschlussvorrichtung (14) aus einem Stück ist, das mittels eines einzigen Strangpress- und Formpressvorgangs hergestellt wird.

15. Reagenzglas gemäß einem der vorhergehenden Ansprüche, **dadurch gekennzeichnet, dass** der Behälterkörper (12) Zentrierungs- und Klemmelemente (40, 140) aufweist, die mit dem Behälterkörper (12) einstückig ausgebildet sind.

16. Reagenzglas gemäß einem der vorhergehenden Ansprüche, **dadurch gekennzeichnet, dass** der Behälterkörper (12) ein einziges Stück ist, das mittels eines einzigen Vorgangs des Strangpressens und Formpressens von Kunststoff-Polymermaterial hergestellt wird, der auch die Herstellung des Saugmittels (13, 115) beinhaltet.

17. Verfahren zum Analysieren flüssiger oder verdünnter biologischer Proben, das nacheinander vorsieht, dass die von einem Patienten genommene Probe in ein Reagenzglas (10) gemäß einem der vorhergehenden Ansprüche unter der Verwendung eines Einführteils (22) des Reagenzglases (10) gegeben wird und das Reagenzglas für die nachfolgende Analyse oder Aufbewahrung mittels einer Verschlussvorrichtung (14) des Reagenzglases (10) abgedichtet wird, wobei das abgedichtete Reagenzglas (10) auch direkt als ein Behälter in der Analyse mittels automatischer Instrumente oder unter der Verwendung manueller Verfahren ohne die Notwendigkeit weiterer Verbringungen der anfänglich vorgesehenen Probe verwendet wird.

## Revendications

1. Tube à essai pour prélever et contenir un échantillon pour des analyses de diagnostic, comprenant :
- un corps contenant (12) doté d'un bord d'extrémité (11a) qui délimite une ouverture (11) ;
- un élément tubulaire (22) faisant saillie vers l'extérieur par rapport audit bord d'extrémité (11a) et relié avec l'intérieur dudit corps contenant (12) par le biais de ladite ouverture (11) pour le passage de l'échantillon ;
- des moyens d'aspiration (13, 115) formant un seul tenant avec ledit corps contenant (12) et pouvant être amenés sélectivement à prélever un volume souhaité d'échantillon par le biais dudit élément tubulaire (22) ;
- un dispositif de fermeture (14) bloqué sur ledit bord d'extrémité (11a) et comprenant un élément de fermeture (18) mobile sélectivement vers une position de fermeture superposée située au-dessus dudit bord d'extrémité (11a) afin de fermer ladite ouverture (11) et un élément de support (16) pour ledit élément tubulaire (22), couplé audit bord d'extrémité (11a) et relié audit élément de fermeture (18), dans lequel, dans ladite position de fermeture superposée, ledit élément de fermeture (18) se conforme de sorte à être couplé audit élément de support (16) de sorte à fermer complètement ledit corps contenant (12),
**caractérisé en ce que** ledit élément de support (16) est relié audit élément de fermeture (18) au moyen d'un élément de liaison flexible (20), configuré pour se déformer lorsque l'élément de fermeture (18) est amené vers ladite position de fermeture pour fermer l'ouverture (11), ledit élément tubulaire (22) étant intercalé entre ledit élément de support (16) et ledit élément de fermeture (18), ledit élément tubulaire (22) étant configuré pour se détacher dudit élément de support (16) lorsque l'élément de fermeture (18) est déplacé d'une position extérieure à la masse du corps contenant (12) vers ladite position de fermeture.

2. Tube à essai selon la revendication 1, **caractérisé en ce que** le détachement de l'élément tubulaire (22) est effectué par désaccouplement.

3. Tube à essai selon la revendication 1, **caractérisé en ce que** ledit élément tubulaire (22) présente au moins une ligne de fragilisation (30) en correspondance avec la région le reliant au dispositif de fermeture (14), qui définit une condition de rupture préférentielle pour le détachement sélectif dudit élément tubulaire (22) dudit dispositif de fermeture (14).

4. Tube à essai selon la revendication 3, **caractérisé en ce que** ladite ligne de fragilisation (30) s'étend le long d'une trajectoire fermée, de sorte à définir un détachement complet de l'élément tubulaire (22) dudit dispositif de fermeture (14).

5. Tube à essai selon la revendication 3 ou 4, **caractérisé en ce que** l'élément tubulaire (22) se couple sur l'élément de support (16) dudit dispositif de fermeture (14) au moyen d'une partie de couplage (25) qui présente ladite au moins une ligne de fragilisation (30) de rupture préférentielle.

6. Tube à essai selon l'une quelconque des revendications précédentes, **caractérisé en ce que** lesdits moyens d'aspiration sont formés par au moins une pièce élastiquement déformable (13, 115), qui se conforme de sorte à être sélectivement comprimée d'une première position inactive à une seconde position déformée, de sorte à expulser un volume d'air déterminé du corps contenant (12) et à s'étendre à nouveau, retournant à ladite première position inactive, de sorte à prélever le volume d'échantillon souhaité par effet d'aspiration.

7. Tube à essai selon la revendication 6, **caractérisé en ce que** ladite pièce élastiquement déformable est formée par au moins une partie déformable d'au moins une paroi latérale (13) ou inférieure dudit corps contenant (12).

8. Tube à essai selon la revendication 6, **caractérisé en ce que** ladite pièce élastiquement déformable est formée par un élément de pompage de type à soufflet (115) qui forme au moins une partie de la partie inférieure dudit corps contenant (12).

9. Tube à essai selon l'une quelconque des revendications précédentes, **caractérisé en ce que** l'élément tubulaire (22) est formé d'un seul tenant avec le dispositif de fermeture (14).

10. Tube à essai selon l'une quelconque des revendications précédentes, **caractérisé en ce que** l'élément tubulaire (22) constitue une pièce séparée par rapport au dispositif de fermeture (14) ou au corps contenant (12) et est couplé de manière sélectivement amovible, par jonction, ancrage ou autre fixation, à une partie associée du dispositif de fermeture (14) ou du corps contenant (12).

11. Tube à essai selon l'une quelconque des revendications précédentes, **caractérisé en ce que** ledit élément tubulaire se présente sous la forme d'une goulotte ou d'une tige d'échantillonnage (22).

12. Tube à essai selon l'une quelconque des revendications précédentes, **caractérisé en ce que** ledit élément de fermeture (18) présente une dent périphérique (19) apte à être entraînée avec un mouvement en porte-à-faux pour l'ouverture/la fermeture.

13. Tube à essai selon l'une quelconque des revendications précédentes, **caractérisé en ce que** ledit dispositif de fermeture (14) est constitué d'un seul tenant, ledit élément tubulaire (22) constituant au moins un prolongement structurel et matériel dudit élément de fermeture (18).

14. Tube à essai selon la revendication 13, **caractérisé en ce que** ledit dispositif de fermeture (14) est une seule pièce fabriquée au moyen d'une seule opération d'extrusion ou de moulage.

15. Tube à essai selon l'une quelconque des revendications précédentes, **caractérisé en ce que** ledit corps contenant (12) inclut des éléments de centrage et de fixation (40, 140) fabriqués d'un seul tenant avec ledit corps contenant (12).

16. Tube à essai selon l'une quelconque des revendications précédentes, **caractérisé en ce que** ledit corps contenant (12) est une seule pièce fabriquée au moyen d'une seule opération d'extrusion et de moulage de matériau polymère plastique, incluant également la fabrication desdits moyens d'aspiration (13, 115).

17. Procédé d'analyse de liquide ou d'échantillons biologiques dilués qui consiste, en séquence, à introduire l'échantillon prélevé chez un patient dans un tube à essai (10) selon l'une quelconque des revendications précédentes, au moyen d'une partie d'introduction (22) du tube à essai (10) et à sceller ledit tube à essai au moyen d'un dispositif de fermeture (14) du tube à essai (10) pour une analyse ou conservation ultérieure, dans lequel ledit tube à essai fermé hermétiquement (10) est également utilisé directement en tant que récipient dans l'analyse au moyen d'instruments automatiques ou au moyen de procédés manuels, sans qu'aucun autre transfert de l'échantillon initial soit nécessaire.
